# EUROPÄISCHE PATENTSCHRIFT

(11) **EP 0 915 838 B1**
(45) Veröffentlichungstag und Bekanntmachung des Hinweises auf die Patenterteilung: **19.09.2001**
(21) Anmeldenummer: 97938853.5
(22) Anmeldetag: 23.07.1997
(51) Int. Cl.: C07C 253/30, C07C 209/60

(54) **VERFAHREN ZUR HERSTELLUNG VON 6-AMINOCAPRONITRIL**
PROCESS FOR PREPARING 6-AMINOCAPRONITRILE
PROCEDE POUR PREPARER DU 6-AMINOCAPRONITRILE

(30) Priorität: 03.08.1996 DE 19631522
(43) Veröffentlichungstag der Anmeldung: 19.05.1999
(73) Patentinhaber: BASF AKTIENGESELLSCHAFT, 67056 Ludwigshafen (DE)
(72) Erfinder: FISCHER, Rolf, D-69121 Heidelberg (DE); PACIELLO, Rocco, D-67098 Bad Dürkheim (DE); RÖPER, Michael, D-67157 Wachenheim (DE); SCHNURR, Werner, D-67273 Herxheim (DE)
(86) Internationale Anmeldenummer: EP9703987
(87) Internationale Veröffentlichungsnummer: WO9805631

(56) Entgegenhaltungen:
- EP-A- 0 011 401
- EP-A- 0 376 121

## Beschreibung

Die vorliegende Anmeldung betrifft ein Verfahren zur Herstellung von 6-Aminocapronitril oder 6-Aminocapronitril-Hexamethylendiamin-Gemischen ausgehend von 5-Formylvaleronitril.

In der EP-A 11401 wird die reduktive Aminierung von δ-Cyanovaleraldehyd zu Hexamethylendiamin beschrieben. Gemäß Beispiel 4 der genannten Anmeldung wurde ein Gemisch mit 60 % δ-Cyanovaleraldehyd mit Ammoniak und Wasserstoff bei einer Temperatur von 100°C und einem Wasserstoffdruck von 140 bar in Gegenwart von Raney-Nickel während zwei Stunden umgesetzt, wobei der Umsatz (bezogen auf die δ-Verbindung) nur 25 % betrug. Der niedrige Umsetzungsgrad zeigt, daß es sich bei der aminierenden Hydrierung einer Aldehydgruppe und der Hydrierung einer Nitrilgruppe im gleichen Molekül zu einem Diamin um ein schwieriges Hydrierproblem handelt. Ferner wird die Bildung von 6-Aminocapronitril nicht beschrieben. Des weiteren ist die Standzeit des verwendeten Katalysators für eine wirtschaftliche Nutzung unbefriedigend.

Aus US-A 2,777,873 ist bekannt, 5-Formylvaleriansäureester mit Ammoniak und Wasserstoff in Gegenwart von Nickel-, Cobalt-, Eisen-, Platin- oder Palladiumkatalysatoren bei 100 bis 160 °C und Drücken von 1 bis 1 000 Atmosphären aminierend zu 6-Aminocapronsäureestern zu hydrieren. In der EP-A 376 121 ist diese Reaktion auch für Rutheniumkatalysatoren beschrieben, wobei man bei Temperaturen im Bereich von 80 bis 140 °C und Drücken im Bereich von 40 bis 1000 bar arbeitet.

Für die Hydrierung von Adipodinitril zu Hexamethylendiamin in Gegenwart von Ammoniak sind nach US-A 3,461,167, Spalte 3, Zeilen 66 bis 74, Cobalt-, Kupfer- und Rheniumkatalysatoren geeignet. Gearbeitet wird bevorzugt bei 70 bis 170 °C und 300 bis 7 000 p.s.i. Nach der US-A 3,471,563 können für diese Umsetzung auch Rutheniumkatalysatoren verwendet werden.

Elemente der achten Nebengruppe hydrieren somit sowohl Nitril- als auch Aldehydgruppen zu Aminogruppen.

Der vorliegenden Erfindung lag daher die Aufgabe zugrunde, ein Verfahren zur Verfügung zu stellen, das es gestattet, ausgehend von 5-Formylvaleraldehyd entweder 6-Aminocapronitril oder ein Gemisch von 6-Aminocapronitril und Hexamethylendiamin mit einem sehr hohen Umsatz herzustellen. Insbesondere war es das Ziel, ein Verfahren zu finden, das lange Verweilzeiten der Katalysatoren garantiert.

Demgemäß wurde ein Verfahren zur Herstellung von 6-Aminocapronitril oder einem Gemisch aus 6-Aminocapronitril und Hexamethylendiamin gefunden, indem man
a) 5-Formylvaleronitril mit Ammoniak und Wasserstoff in Gegenwart von Hydrierkatalysatoren, ausgewählt aus der Gruppe, bestehend aus Metallen oder Metallverbindungen von Cobalt, Ruthenium und Palladium, umsetzt unter Erhalt eines Hydrieraustrages, und
b) aus dem Hydrieraustrag 6-Aminocapronitril und gegebenenfalls Hexamethylendiamin gewinnt.

Als Ausgangsverbindung setzt man erfindungsgemäß 5-Formylvaleraldehyd ein. Aus der Patentliteratur ist eine Reihe von Möglichkeiten zur Herstellung von 5-Formylvaleronitril bekannt:

In der WO 94/26688 wird ein Verfahren beschrieben, bei dem
(a) interne substituierte Olefine zu endständigen Olefinen isomerisiert,
(b) die endständigen Olefine in Gegenwart der internen Olefine bevorzugt hydroformyliert,
(c) die Produkte der Hydroformylierung abgetrennt und
(d) die internen Olefine in die Isomerisierung zurückgeführt werden.

In Anspruch 3 der genannten WO 94/26688 werden nitrilhaltige Olefine beansprucht. Als Hydroformylierungs-Katalysatoren werden Rhodium-Triphenylphosphin-Systeme verwendet, in denen das Triphenylphosphin durch geeignete funktionelle Gruppen wasserlöslich gemacht wird.

In der WO 95/18783 wird die Hydroformylierung von internen nitrilhaltigen Olefinen mit wasserlöslichen Platinkatalysatoren beschrieben.

Aus der EP-A 11401 ist ferner bekannt, 3-Pentennitril mit Kohlenmonoxid und Wasserstoff unter Druck in Gegenwart eines Cobalt-Katalysators umzusetzen. Dabei entsteht ein Gemisch aus isomeren Formylvaleronitrilen und den der Aldehydgruppe entsprechenden Alkoholen.

Erfindungsgemäß setzt man 5-Formylvaleronitril bei Temperaturen im Bereich von 40°C bis 150°C, vorteilhaft von 50°C bis 140°C, insbesondere von 60°C bis 130°C, und Drücken im Bereich von 2 bis 350, vorteilhaft von 20 bis 300 bar, insbesondere von 40 bis 250 bar, mit Ammoniak und Wasserstoff in Gegenwart von Hydrierkatalysatoren in einem ersten Schritt (Stufe a)) unter Erhalt eines Hydrieraustrages um.

Die Umsetzung führt man bevorzugt in flüssigem Ammoniak als Lösungsmittel durch, wobei der Ammoniak gleichzeitig als Reaktionspartner dient. Die Ammoniak-Menge beträgt in der Regel 1 bis 80 Mol, insbesondere 10 bis 50 Mol Ammoniak pro Mol 5-Formylvaleronitril. Es kann auch vorteilhaft sein, zusätzlich zu Ammoniak ein unter den Reaktionsbedingungen inertes Lösungsmittel, wie z. B. Alkohole, Ester, Ether, Kohlenwasserstoffe mitzuverwenden, wobei man im allgemeinen ein Gewichtsverhältnis von Lösungsmittel zu 5-Formylvaleronitril im Bereich von 0,1 : 1 bis 5 : 1, vorzugsweise von 0,5 :1 bis 3 : 1, wählt. Besonders bevorzugt sind Alkohole wie Methanol und Ethanol.

Die Menge an Wasserstoff wählt man üblicherweise so, daß das Molverhältnis von Wasserstoff zu 5-Formylvaleronitril im Bereich von 1 : 1 bis 100 : 1, insbesondere von 5 : 1 bis 50 : 1 liegt.

Als Katalysatoren setzt man erfindungsgemäß Hydrierkatalysatoren ein, die ausgewählt sind aus der Gruppe, bestehend aus Metallen oder Metallverbindungen von Cobalt, Ruthenium und Palladium.

Bei den erfindungsgemäß einsetzbaren Katalysatoren kann es sich um Voll- oder Trägerkatalysatoren handeln. Als Trägermaterialien kommen beispielsweise poröse Oxide wie Aluminiumoxid, Siliciumdioxid, Alumosilikate, Lanthanoxid, Titandioxid, Zirkondioxid, Magnesiumoxid, Zinkoxid und Zeolithe sowie Aktivkohle oder Mischungen der genannten Verbindungen in Betracht.

Die Katalysatoren können als Festbettkatalysatoren in Sumpf- oder Rieselfahrweise oder als Suspensionskatalysatoren eingesetzt werden. Dabei wählt man bevorzugt eine Katalysatorbelastung im Bereich von 0,1 bis 2,0, vorzugsweise von 0,3 bis 1 kg 5-Formylvaleronitril/l Katalysator • Stunde.

Es ist auch möglich, Verbindungen der oben genannten Metalle als homogen gelöste Hydrierkatalysatoren zu verwenden.

In einer bevorzugten Ausführungsform können die oben genannten Katalysatoren weiterhin 0,01 bis 25, vorzugsweise 0,1 bis 5 Gew.-%, bezogen auf die Gesamtmenge an Hydriermetallen (berechnet als Elemente) , mindestens eines Promotors auf der Basis eines Metalls, ausgewählt aus der Gruppe Kupfer, Silber, Gold, Mangan, Zink, Cadmium, Blei, Zinn, Scandium, Yttrium, Lanthan und der Lanthanidelemente, Titan, Zirkon, Hafnium, Chrom, Molybdän, Wolfram, Vanadium, Tantal, Antimon, Bismuth, Aluminium enthalten, sowie ferner durch 0,01 bis 5, vorzugsweise 0,1 bis 3 Gew.-%, bezogen auf die Hydriermetalle (berechnet als Elemente), einer Verbindung auf der Basis eines Alkalimetalles oder eines Erdalkalimetalles dotiert sein, vorzugsweise Alkalimetall- und Erdalkalihydroxide wie Lithiumhydroxid, Natriumhydroxid, Kaliumhydroxid, Rubidiumhydroxid, Cäsiumhydroxid, besonders bevorzugt Lithiumhydroxid.

Die im erfindungsgemäßen Verfahren eingesetzten Katalysatoren können z. B. sogenannte Fällungskatalysatoren sein. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder Carbonat-Lösungen, z. B. schwerlösliche Hydroxide, Oxidhydrate, basische Salze oder Carbonate ausfällt, die erhaltenen Niederschläge anschließend trocknet und diese dann durch Calcinierung bei Temperaturen im Bereich von im allgemeinen 300 bis 700°C, insbesondere von 400 bis 600°C in die entsprechenden Oxide, Mischoxide und/oder gemischtvalentigen Oxide umwandelt, welche in der Regel durch eine Behandlung mit Wasserstoff oder mit Wasserstoff enthaltenden Gasen bei üblicherweise 50 bis 700°C, insbesondere 100 bis 400°C, zu den betreffenden Metallen und/oder oxidischen Verbindungen niederer Oxidationsstufe reduziert, und dabei in die eigentliche, katalytisch aktive Form überführt werden. Hierbei wird in der Regel so lange reduziert, bis kein Wasser mehr gebildet wird.

Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft aber auch gleichzeitig mit dem Trägermaterial aus den betreffenden Salzlösungen gefällt werden. Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten. Außer den oben genannten Fällungskatalysatoren, welche außer den katalytisch aktiven Komponenten noch zusätzlich ein Trägermaterial enthalten, eignen sich für das erfindungsgemäße Verfahren im allgemeinen solche Trägermaterialien, bei denen die katalytisch-hydrierend wirkende Komponenten, z. B. durch Imprägnierung, auf ein Trägermaterial aufgebracht worden sind.

Die Art der Aufbringung der katalytisch aktiven Metalle auf den Träger ist in der Regel nicht kritisch und kann auf verschiedenerlei Art und Weise bewerkstelligt werden. Die katalytisch aktiven Metalle können auf diesen Trägermaterialien z.B. durch Tränkung mit Lösungen oder Suspensionen der Salze oder Oxide der betreffenden Elemente, Trocknung und anschließende Reduktion der Metallverbindungen zu den betreffenden Metallen oder Verbindungen niederer Oxidationsstufe mittels eines Reduktionsmittels, vorzugsweise mit Wasserstoff oder komplexen Hydriden, aufgebracht werden.

Eine andere Möglichkeit zur Aufbringung der katalytisch aktiven Metalle auf diese Träger besteht darin, die Träger mit Lösungen thermisch leicht zersetzbarer Salze, z. B. mit Nitraten oder thermisch leicht zersetzbaren Komplexverbindungen, z. B. Carbonyl- oder Hydrido-Komplexen der katalytisch aktiven Metalle, zu imprägnieren und den so getränkten Träger zwecks thermischer Zersetzung der adsorbierten Metallverbindungen auf Temperaturen im Bereich von in der Regel 300 bis 600°C zu erhitzen. Diese thermische Zersetzung wird vorzugsweise unter einer Schutzgasatmosphäre vorgenommen. Geeignete Schutzgase können beispielsweise Stickstoff, Kohlendioxid, Wasserstoff oder ein Edelgas sein.

Weiterhin können die katalytisch aktiven Metalle auf dem Katalysatorträger durch Aufdampfen oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Es versteht sich für den Fachmann von selbst, daß höhere Gehalte dieser Trägerkatalysatoren an katalytisch aktiven Metallen üblicherweise zu höheren Raum-Zeit-Umsätzen führen können als niedrigere Gehalte. Im allgemeinen werden Trägerkatalysatoren verwendet, deren Gehalt an katalytisch aktiven Metallen 0,1 bis 90 Gew.-%, vorzugsweise 0,5 bis 40 Gew.-%, bezogen auf den gesamten Katalysator beträgt.

Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, können diese Angaben nach bisherigen Beobachtungen aber auch unter- oder überschritten werden, ohne daß sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle beispielsweise nach dem Verfahren von DE-A 2 519 817, EP-A 1 477 219 und EP-A 285 420 auf den Träger aufgebracht werden. In den Katalysatoren gemäß den vorgenannten Schriften liegen die katalytisch aktiven Metalle als Legierung vor, die durch thermische Behandlung und/oder Reduktion der z. B. durch Tränkung mit einem Salz oder Komplex der zuvor genannten Metalle erzeugt werden können.

Sowohl die Aktivierung der Fällungskatalysatoren als auch der Trägerkatalysatoren kann auch in situ zu Beginn der Reaktion durch den anwesenden Wasserstoff erfolgen, bevorzugt werden diese Katalysatoren jedcch vor ihrer Verwendung separat aktiviert.

Aus dem in Stufe a) des erfindungsgemäßen Verfahrens erhaltenen Hydrieraustrag gewinnt man nach üblichen Methoden wie Destillation 6-Aminocapronitril, gegebenenfalls zusammen mit Hexamethylendiamin (Stufe b)).

In einer bevorzugten Ausführungsform trennt man vor der Gewinnung von 6-Aminocapronitril und gewünschtenfalls Hexamethylendiamin in Stufe b) überschüssigen Ammoniak, Wasserstoff und gewünschtenfalls den Katalysator ab.

In einer weiteren bevorzugten Ausführungsform behandelt man 5-Formylvaleronitril zunächst bei Temperaturen im Bereich 40 bis 150°C mit Ammoniak (erste Stufe) unter Erhalt eines ammoniakalischen Austrags. Dies kann beispielsweise in einem Vorreaktor erfolgen. Diese Umsetzung kann in Abwesenheit oder aber bevorzugt in Anwesenheit eines sauren, homogenen oder heterogenen Katalysators erfolgen. Die Katalysator-Belastung beträgt dabei (im Falle von heterogenen Katalysatoren) üblicherweise 0,1 bis 2,0 kg 5-Formylvaleronitril/l Katalysator • Stunde.

Der ammoniakalische Austrag kann dann gewünschtenfalls vom sauren Katalysator befreit werden (zweite Stufe).

In einer dritten Stufe setzt man den ammoniakalischen Austrag bzw. die ammoniakalische Lösung mit Ammoniak und Wasserstoff in Gegenwart von Hydrierkatalysatoren, ausgewählt aus der Gruppe, bestehend aus Metallen oder Metallverbindungen der Elemente Kupfer, Rhenium und Elementen der achten Nebengruppe, um unter Erhalt eines Hydrieraustrags, wobei das Verfahren in der Regel wie das weiter oben beschriebene Verfahren durchgeführt wird. Im Anschluß daran gewinnt man in an sich bekannter Art und Weise 6-Aminocapronitril und gegebenenfalls Hexamethylendiamin aus dem Hydrieraustrag.

Als saure Katalysatoren kann man beispielsweise Zeolithe in der H-Form, saure Ionentauscher, Heteropolysäuren, saure und supersaure Metalloxide, die gegebenenfalls mit Sulfat oder Phosphat dotiert wurden und anorganische oder organische Säuren, verwenden.

Als Zeolithe eigenen sich beispielsweise Vertreter aus der Mordenit-Gruppe oder engporige Zeolithe vom Erionit- bzw. Chabasit-Typ oder Zeolithe von Faujasit-Typ, z. B. Y-, X- oder L-Zeolithe. In diese Gruppe gehören auch die sogenannten "ultrastabilen" Zeolithe des Faujasittyps, d. h. dealuminierte Zeolithe.

Besonders vorteilhaft unter den Zeolithen sind solche mit Pentasilstruktur wie ZSM-5, ZSM-11 und ZBM-10. Diese haben als Grundbaustein einen aus SiO₂-Tetraedern aufgebauten Fünfring gemeinsam. Sie sind durch ein hohes SiO₂/Al₂O₃-Verhältnis gekennzeichnet sowie durch Porengrößen, die zwischen denen der Zeolithe vom Typ A und denen vom Typ X oder Y liegen.

Die erfindungsgemäß eingesetzten Heteropolysäuren sind anorganische Polysäuren, die im Gegensatz zu Isopolysäuren mindestens zwei verschiedene Zentralatome besitzen. Als Beispiele seien Dodecawolframphosphorsäure H₃PW₁₂O₄₀ • xH₂O, Dodecamolybdophosphorsäure H₃PMO₁₂O₄₀ • xH₂O genannt. Prinzipiell können die in EP-A 158 229 genannten Katalysatoren und Katalysatorkombinationen eingesetzt werden.

Bevorzugte Heteropolysäuren sind Heteropolysäuren von Molybdän oder Wolfram mit Phosphorsäure, Tellursäure, Selensäure, Arsensäure, Kieselsäure, insbesondere mit Phosphorsäure.

Die Protonen der Heteropolysäuren können teilweise durch Metallionen ersetzt sein, bevorzugt sind dabei die Alkali- und Erdalkalimetallionen.

Bevorzugte saure Ionentauscher sind z. B. vernetzte Polystyrole mit Sulfonsäuregruppen.

Saure Metalloxide sind beispielsweise SiO₂, Al₂O₃, ZrO₂, Ga₂O₃, PbO₂, Sc₂O₃, La₂O₃, TiO₂, SnO₂ usw. oder Kombinationen einzelner Oxide. Die Oxide können zur Säurestärkeerhöhung auch mit Mineralsäuren wie z. B. Schwefelsäure behandelt werden.

Als Säuren sind belspielsweise Mineralsäuren wie Schwefelsäure und Phosphorsäure sowie organische Säuren, wie z. B. Sulfonsäuren geeignet.

Als supersaure Metalloxide sind z. B. Sulfat-dotiertes ZrO₂ oder Molybdän- oder Wolfram enthaltendes ZrO₂ geeignet.

In einer weiteren bevorzugten Ausführungsform führt man die Hydrierung an einem Hydriermetall, aufgebracht auf einem der genannten oxidischen Träger, durch. Nach Entfernung von überschüssigem Wasserstoff und gegebenenfalls des Katalystors wird der Hydrieraustrag bevorzugt durch fraktionierende Destillation zu 6-Aminocapronitril und gegebenenfalls Hexamethylendiamin aufgearbeitet.

Nach dem erfindungsgemäßen Verfahren erhält man 6-Aminocapronitril mit sehr guten Umsätzen, guten Ausbeuten und Selektivitäten. Es ist auch möglich, durch Variation von Temperatur und Katalysatorbelastung Gemische aus 6-Aminocapronitril und Hexamethylendiamin zu erhalten. Dabei begünstigen höhere Temperaturen und niedrige Katalysatorbelastungen die Bildung von Hexamethylendiamin, niedrigere Temperaturen und hohe Katalysatorbelastungen die Bildung von 6-Aminocapronitril.

6-Aminocapronitril und Hexamethylendiamin stellen wichtige Faservorprodukte dar. 6-Amino-capronitril läßt sich zu Caprolactam, dem Vorprodukt für die Herstellung von Nylon 6, cyclisieren. Hexamethylendiamin wird hauptsächlich mit Adipinsäure zu dem sogenannten AH-Salz, der Vorstufe von Nylon 6.6, umgesetzt.

### Beispiele

### Beispiel 1

In einem 300 ml Autoklaven mit Probenahmeschleuse (Werkstoff HC 4) wurden 11 g 5-Formylvaleronitril und 3 g Ru (3 %)/Al₂O₃ (4 mm-Stränge) unter Schutzgas (Argon) eingefüllt. Anschließend wurde der Autoklav verschlossen und 150 ml NH₃ wurden aufgepreßt. Die Durchmischung erfolgte mit einem Magnetrührer. Nach Erhitzen auf 80 °C (Eigendruck: ca. 39 bar) wurde das Gemisch noch 2 Stunden bei 80 °C gehalten und dann der Gesamtdruck durch Wasserstoff auf 70 bar erhöht. Der Druck von 70 bar wurde durch ständiges Nachpressen von Wasserstoff gehalten. Nach 25 Stunden wurde der Autoklav entspannt und der Hydrieraustrag gaschromatographisch analysiert. Als Produkte entstanden 73 % 6-Aminocapronitril und 12 % Hexamethylendiamin. Der Umsatz betrug 100 %.

### Beispiel 2

In dem in Beispiel 1 beschriebenen Autoklaven wurden 20 g 5-Formylvaleronitril und 3 g Pd (5 %)/ Al₂O₃-Pulver und 0,1 g Lithiumhydroxid unter Schutzgas (Argon) eingefüllt. Anschließend wurde der Autoklav geschlossen und 140 ml NH₃ wurden aufgepreßt. Unter Rühren mit einem Magnetrührer wurde auf 100 °C aufgeheizt und der Gesamtdruck durch Aufpressen von Wasserstoff auf 80 bar eingestellt und durch Nachpressen von Wasserstoff auf diesem Wert gehalten. Nach 23 Stunden wurde der Autoklav entspannt und der Hydrieraustrag gaschromatographisch analysiert. Als Produkte entstanden 59,1 % 6-Aminocapronitril und 5,1 % Hexamethylendiamin (Umsatz 100 %).

### Beispiel 3

Der in diesem Beispiel verwendete Cobalt-Katalysator (23 % Co/Al₂O₃, 4 mm Stränge) wurde vor seiner Verwendung für die Herstellung von 6-Aminocapronitril 2 Stunden lang bei 250 °C unter strömendem Wasserstoff aktiviert.

In dem in Beispiel 1 beschriebenen Autoklaven wurden 32 g 5-Formylvaleronitril und 10 g Cobalt-Katalysator unter Argon eingefüllt. Anschließend wurde der Autoklav geschlossen und 130 ml Ammoniak wurden aufgepreßt. Unter Rühren mit einem Magnetrührer wurde auf 100 °C aufgeheizt und der Gesamtdruck durch Aufpressen von Wasserstoff auf 100 bar eingestellt und durch Nachpressen von Wasserstoff, auf diesem Wert gehalten. Nach 20 Stunden wurde der Autoklav entspannt und der Hydrieraustrag gaschromatographisch analysiert. Als Reaktionsprodukte wurden 56 % 6-Aminocapronitril und 6 % Hexamethylendiamin (Umsatz 100 %) gefunden.

## Patentansprüche

1. Verfahren zur Herstellung von 6-Aminocapronitril oder 6-Aminocapronitril/Hexamethylendiamin-Gemischen, **dadurch gekennzeichnet, daß** man
a) 5-Formylvaleronitril mit Ammoniak und Wasserstoff in Gegenwart von Hydrierkatalysatoren, ausgewählt aus der Gruppe, bestehend aus Metallen oder Metallverbindungen von Cobalt, Ruthenium und Palladium, umsetzt unter Erhalt eines Hydrieraustrages und
b) aus dem Hydrieraustrag 6-Aminocapronitril und gegebenenfalls Hexamethylendiamin gewinnt.

2. Verfahren nach Anspruch 1, **dadurch gekennzeichnet, daß** man vor der Gewinnung von 6-Aminocapronitril und gegebenenfalls Hexamethylendiamin in Stufe b) überschüssigen Ammoniak und Wasserstoff abtrennt.

3. Verfahren nach Anspruch 2, **dadurch gekennzeichnet, daß** man in Stufe b) zusätzlich den Katalysator abtrennt.

4. Verfahren nach den Ansprüchen 1 bis 3, wobei man vor Schritt a) 5-Formylvaleronitril zunächst mit Ammoniak behandelt unter Erhalt eines ammoniakalischen Austrags und den ammoniakalischen Austrag in Schritt a) einsetzt.

5. Verfahren nach Anspruch 4, wobei man die Umsetzung vor Schritt a) zusätzlich in Gegenwart eines sauren Katalysators durchführt.

6. Verfahren nach Anspruch 5, wobei man vor Schritt a) den Katalysator von dem ammoniakalischen Austrag abtrennt.

## Claims

1. A process for the preparation of 6-aminocapronitrile or a 6-aminocapronitrile/hexamethylene diamine mixture, wherein
a) 5-formylvaleronitrile is caused to react with ammonia and hydrogen in the presence of a hydrogenation catalyst selected from the group consisting of metals or metal compounds of cobalt, ruthenium and palladium, giving a hydrogenation effluent, and
b) 6-aminocapronitrile and possibly hexamethylene diamine is/are isolated from the hydrogenation effluent.

2. A process as defined in claim 1, wherein excess ammonia and hydrogen are separated off prior to the isolation of 6-aminocapronitrile and possibly hexamethylene diamine in stage b).

3. A process as defined in claim 2, wherein the catalyst is additionally separated off in stage b).

4. A process as defined in any of claims 1 to 3, where prior to step a) 5-formylvaleronitrile is first of all treated with ammonia to give an ammoniacal effluent and the ammoniacal effluent is used in step a).

5. A process as defined in claim 4, where the reaction is additionally carried out in the presence of an acidic catalyst prior to step a).

6. A process as defined in claim 5, where the catalyst is separated off from the ammoniacal effluent prior to step a).

## Revendications

1. Procédé de préparation de 6-aminocapronitrile ou de mélanges de 6-aminocapronitrile/hexaméthylènediamine, **caractérisé en ce que**:
a) on fait réagir du 5-formylvaléronitrile avec de l'ammoniac et de l'hydrogène en présence de catalyseurs d'hydrogénation, choisis dans le groupe formé par des métaux ou des composés métalliques de cobalt, ruthénium et palladium, avec obtention d'un produit d'hydrogénation, et
b) on obtient à partir du produit d'hydrogénation du 6-aminocapronitrile et éventuellement de l'hexaméthylènediamine.

2. Procédé selon la revendication 1, **caractérisé en ce que**, avant l'obtention de 6-aminocapronitrile et éventuellement d'hexaméthylènediamine à l'étape b), on sépare l'ammoniac et l'hydrogène en excès.

3. Procédé selon la revendication 2, **caractérisé en ce qu'**à l'étape b) on sépare également le catalyseur.

4. Procédé selon les revendications 1 à 3, dans lequel, avant l'étape a), on traite d'abord le 5-formylvaléronitrile par de l'ammoniac avec obtention d'un produit ammoniacal, et que l'on met en oeuvre ledit produit ammoniacal dans l'étape a).

5. Procédé selon la revendication 4, où la réaction précédant l'étape a) est en outre entreprise en présence d'un catalyseur acide.

6. Procédé selon la revendication 5, où le catalyseur est séparé du produit ammoniacal avant l'étape a).
